# EUROPEAN PATENT SPECIFICATION

(11) **EP 2 307 559 B1**
(45) Date of publication and mention of the grant of the patent: **02.09.2015**
(21) Application number: 09716839.7
(22) Date of filing: 03.03.2009
(51) Int. Cl.: C12Q 1/04, G01N 33/50, G01N 33/49

(54) **INFECTION MEDIATED FOAM DISSOLUTION RATE MEASUREMENT**
INFEKTIONSVERMITTELTE SCHAUMAUFLÖSUNGSGESCHWINDIGKEITSMESSUNG
MESURE DE LA VITESSE DE DISSOLUTION D' UNE MOUSSE MÉDIÉE PAR UNE INFECTION

(30) Priority: 03.03.2008 US 33166 P
(43) Date of publication of application: 13.04.2011
(73) Proprietor: Imigene, Inc., Madeira Beach, FL 33708 (US)
(72) Inventor: EWERT, Matt, St. Petersburg, FL 33708 (US)
(74) Representative: Stevens, Fiona
(86) International application number: PCT/US2009/035899
(87) International publication number: WO 2009/111485

(56) References cited:
- TSENG C H ET AL: "NMR of Laser-Polarized<129>Xe in Blood Foam" JOURNAL OF MAGNETIC RESONANCE, ACADEMIC PRESS, ORLANDO, FL, US, vol. 126, no. 1, 1 May 1997 (1997-05-01), pages 79-86, XP004407956 ISSN: 1090-7807 cited in the application
- PATEL P D ET AL: "WHIPPING TEST FOR THE DETERMINATION OF FOAMING CAPACITY OF PROTEIN A COLLABORATIVE STUDY" INTERNATIONAL JOURNAL OF FOOD SCIENCE AND TECHNOLOGY, vol. 23, no. 1, 1988, pages 57-64, XP002534407 ISSN: 0950-5423

## Description

### BACKGROUND OF THE INVENTION

Recognition of sepsis is problematic in the clinical setting. Laboratory tools for monitoring bacterial or fungal infection and response to treatment are currently laboratory based often requiring a centrifuge and labile reagents hence requiring a longer time to result than a state of medical emergency ideally would allow (Hussain *et al*., 2008; Zakariah, 2008). Most markers studied are components of the inflammatory response system and by themselves fail to provide widely acceptable diagnostic solutions (St. Louis, 2007). While sepsis biomarker panels can be made useful through scoring strategies, clinicians need rapid clear inexpensive point of care results.

Patel et al (International Journal of Food Science and Technology, (1988), vol. 23, no. 1, pages 57-64) teaches a standardized whipping test for determining the functional properties of proteins by beating a protein solution, such as a sodium caseinate, bovine serum albumin, whey protein isolate, or albumen solution, in a food mixer to prepare foam.

### BRIEF SUMMARY OF THE INVENTION

The subject invention concerns methods and materials for determining the presence or absence of bacterial or fungal infection in a blood sample. In one embodiment, a method of the invention comprises exposing an anticoagulant treated blood sample from a person or animal to freezing the sample to a solid, followed by thawing of the sample and then agitation of the sample to develop foam and then observing the rate that foam dissolves. The presence of clinical bacterial or fungal infection produces more stable foam compared to a blood sample having an absence of bacterial or fungal infection, which produces less stable foam when agitated.

### DETAILED DESCRIPTION OF THE INVENTION

The subject invention concerns methods and materials for determining the presence or absence of bacterial or fungal infection in a person or animal using a blood sample. In one embodiment, a method of the invention comprises exposing an anticoagulant treated blood sample from a person or animal to freezing the sample to a solid, followed by thawing of the sample and then agitation of the sample to develop foam and then observing the rate that foam dissolves. The presence of clinical bacterial or fungal infection produces more stable foam compared to a blood sample having an absence of bacterial or fungal infection, which produces less stable foam when agitated.

In one embodiment, a method of the present invention comprises determining the presence or absence of bacterial or fungal infection in a person or animal by exposing an anticoagulant treated blood sample to freezing, followed by warming or thawing, and tehn shaking or agitation to develop foam and then observing the rate that the foam boundary dissolves or dissipates. The warming or thawing step can be accomplished in any suitable manner, including, for example, allowing the frozen sample to thaw at room temperature or by placing the sample in a heated water bath. In an exemplified embodiment, the frozen sample is diluted in a liquid solution, such as water or a saline solution. In a further embodiment, a liquid solution is added to the blood sample before the blood sample is frozen. In one embodiment, the anticoagulant is ethylenediaminetetraacetic acid (EDTA), sodium polyanetholesulfonate, citrate, or heparin. In one embodiment, the blood sample is frozen to a solid phase by exposure of the blood sample to a temperature of 0° C or less. In a specific embodiment, the blood sample is exposed to a temperature of 0° to -3.9° C until frozen. In a further embodiment, the blood sample is exposed to a temperature of -4° C or below until frozen. In one embodiment of the method, the foam boundary is detected by a spectrophotometric device. In another embodiment, the foam boundary is detected by electrical current sensors placed in the sample tube. In a still further embodiment, the foam boundary is detected visually or acoustically. In another embodiment, the sample is agitated inside a syringe. In one embodiment, the sample is agitated inside a vacuum tube.

In certain embodiments, the blood from patients who are infected by bacteria or fungus as evidenced by culture will have a foam migration boundary that will migrate between approximately 30 seconds to 10 minutes ahead of the foam migration boundary observed in blood from patients that are not infected. After approximately 15 minutes the foam boundaries will reach approximately the same place in the sample tube. The samples may be shaken again to repeat the test. The foam boundary can be detected visually, acoustically, by electrical current sensors, or optically for instance by a spectrophotometric device.

The subject invention also concerns a method for determining the presence or absence of bacterial or fungal infection in a person or animal by sequentially a) exposing an anticoagulant treated blood sample from the person or animal to freezing below 0° C, b) thawing to a liquid phase and c) determining the level of aggregation of DNA with fibrin.

The subject invention also concerns methods for monitoring the status of an infection in a person or animal over a period of time. In one embodiment, a blood sample from a person or animal is assayed for the presence or absence of infection using the foam assay of the present invention described herein. At a subsequent time period (e.g., 12 to 24 hours later), another blood sample is obtained and assayed for the presence of an infection using the methods of the present invention. The results of the assay can be analyzed to determine whether the infection in the person or animal is persisting or if it is subsiding. Persons or animals that are recovering from infection show, over the period of time that the blood samples are analyzed from the person or animal, an increasing dissolution rate of the foam in the assay of the invention *(i.e.,* the foam dissipates more rapidly). If the person or animal is not recovering from the infection or if the infection is getting worse, then the person or animal will show, over the time period the assay is conducted, a dissolution rate of foam that remains about the same or that even decreases (*i*.*e*., the foam dissipates more slowly) in the assay of the invention. In one embodiment, the assay of the invention is conducted one or two times a day for several days and the rate of foam dissolution compared for each time point tested over the several days.

The present invention is based on the discovery that the presence of clinical bacterial or fungal infection in the blood of a person or animal causes an alteration in the coagulation associated components present in the blood. When a suboptimal level of saponin is used, which can lyse most all of the red blood cells found in a sample of healthy blood, the red blood cells found in samples taken from infected patients showed a resistance to lyses. Addition of plasminogen and streptokinase can eliminate the effect.

There is a need to provide clinicians with a rapid inexpensive presence/absence test for bacterial or fungal infection. As described herein, the biochemical differences between blood from infected and non-infected people can be exaggerated by cooling or freezing the blood in order to promote aggregation of blood elements. This aggregation results in differences in the rate of foam dissolution after the blood sample is shaken.

An important aspect of the present invention is the Foam Migration Assay (FMA). The FMA contributes diagnostic value by letting clinicians know within 15 minutes whether observed symptoms are associated with bacterial/fungal infection or not. The present invention is based on a unique way of measuring disseminated intravascular coagulation (DIC) associated with sepsis. The way the present invention detects DIC has never been described before.

When blood is frozen, DNA is released from white blood cell nuclei, red and white blood cell membranes fracture into fragments and all these elements combine assiduously with fibrin. When blood is agitated, foam is generated mostly from soluble plasma proteins. The freezing induced hemolysis that is a first step of the method of the subject invention liberates phospholipid fragments from the red/white blood cell membranes. These phospholipids act as a surfactant which destabilizes foam formed from during agitation. When DNA and fibrin form an aggregate, phospholipid fragments are trapped (because the fibrin is molecularly linked to red blood cell membranes). The surfactant action of phospholipid is reduced and hence a more stable foam is produced after agitation.

After freezing, thawing, and agitation of the blood sample, the presence of clinical bacterial or fungal infection produces more stable foam compared to a blood sample having an absence of bacterial or fungal infection, which produces less stable foam when agitated. Omission of the freezing step negates this difference. The differences described herein between the blood of infected and non infected patients can also be eliminated by breaking up the fibrin/DNA/phospholipid interaction through addition of various enzymes to EDTA treated blood. For example, one may add either 4,000 Kunitz Units of DNase, 20 U of Plasminogen activated with 10,000 U Streptokinase, or a combination of 2 mg synthetic melittin (a 26 amino acid peptide which stimulates phospholipase A2) and 1,000 U phospholipase A2 from bee venom to a 2 ml EDTA blood sample with 30 mM magnesium chloride. Any one of these enzyme treatments can make the foam derived from agitating frozen blood of infected and non infected patients to destabilize at the same rate and remove the diagnostic differences methods of the subject invention exploit. The enhancement of this interaction via freezing and subsequent spectrophotometric interrogation of this specific molecular interaction as a function of the impact it has on foam stability while the foam dissolves as a measure of sepsis related DIC has never before been reported.

The foam produced by agitating previously frozen blood in the present method can be analyzed spectrophotometrically while the foam dissolves. The present methods become a measurement of how a DIC-related increase in fibrin deposition on red blood cell surfaces, in combination with DNA, traps phospholipid which otherwise would act as a surfactant and destabilize the foam being measured. The present invention is based on measuring macromolecular interactions after the sample is drawn where those interactions are based on cellular and physiological conditions present before the sample was drawn.

Other foam measurement tests have been described. The shake and click test is an inexpensive biophysical test of lung surfactant function, using 0.2 mL samples of tracheal (TA) and gastric aspirates (GA) shaken with 95% ethanol. In this case a reagent is added to indicate the presence of phospholipids. The methods of the subject invention use the blood itself as the reagent input and then change the macromolecular architecture to develop a diagnostic finding.

Tseng *et al*. developed enhanced measurement of hyperpolarized ¹²⁹Xe by using a blood foam (NMR of laser polarized 129Xe in blood foam; Tseng *et al*., 1997). This work involves biochemical pathways and analysis methods different from that employed in the subject invention. U.S. Patent No. 4,425,427, entitled "Simultaneous, kinetic, spectrophotometric analysis of blood serum for multiple components", describes use of a fraction of blood generated by centrifugation and removal of the supernatant. Serum is the preferred input for most all sepsis biomarker tests. When specrophotometry is classically used to detect general disease in whole blood, the blood cells are preserved as an important requirement. The methods of the present invention, however, are based on the destruction of blood cells and the subsequent interaction of the liberated fragments. Prata *et al.* (2008) describes in detail blood plasma foam stability measurements used for quality analysis in the agricultural sector. None of these procedures however involve the whole blood elements that interact to serve as the basis for the subject invention. What none of the above authors describe is a method where whole blood is frozen, diluted, then agitated to produce foam and where the influence of DIC is measured based on spectrophotometric analysis of the foam while it dissolves.

According to the invention, a method for determining the presence or absence of a bacterial or fungal infection in a person or animal by sequentially a) exposing an anticoagulant-treated blood sample of the person or animal to a freezing temperature in a transparent container wherein the blood is frozen to a solid phase, b) thawing the frozen blood sample to a liquid phase, c) mixing by movement of an object that is immersed in the blood sample in order to develop foam, and d) measuring the rate that the foam dissolves or dissipates by illuminating one side of the transparent blood sample container and measuring the rate foam dissolves or dissipates by capturing transmitted light on the other side of the blood sample container using a collimating lens, wherein the diameter of the collimating lens is at least equal to half of the vertical path traveled by the foam liquid boundary as the foam dissolves or dissipates.

In a preferred embodiment, in the method described above, the plane transecting the path of both the light from the illumination source and the light entering the collimating lens is tilted at an angle that is greater or less than 90° to the plane of the blood sample container wall.

In a preferred embodiment, in the method described above, the radius of the collimating lens is at least half the length of the sample container which is occupied by the liquid phase of the blood sample.

In a preferred embodiment, in the method described above, the illumination source and the light collecting collimating lens which is attached to a light detector are tilted at an angle that is greater or less than 90° relative to the plane of the blood sample container.

The patient being tested for infection can be a human or other mammal, such as a primate (monkey, chimpanzee, ape, *etc*.), dog, cat, cow, pig, or horse, or other animal infected with a bacteria or fungus. Bacterial infections that can be detected according to the present invention include, but are not limited to, those from *Staphylococcus, Streptococcus, Salmonella, Bacillus, Clostridium, Pseudomonas, Neisseria*, *Mycobacterium*, *Yersinia*, *Haemophilus, Bordetella*, *Listeria*, *Treponema*, *Chlamydia*, *Brucella,* and *Vibrio.* Fungal infections that can be detected according to the present invention include, but are not limited to, fungi of the genus *Histoplasma (e.g., Histoplasma capoulatum), Coccidioides (e.g., C*. *immitis), Blastomyces (e.g., B*. *dermatitidis), Candida*, *Aspergillus*, *Cryptococcus,* and *Zygomycetes.*

The methods of the present invention can be used to detect the presence or absence of infection in humans and other animals. The other animals contemplated within the scope of the invention include domesticated, agricultural, or zoo- or circus-maintained animals. Domesticated animals include, for example, dogs, cats, rabbits, ferrets, guinea pigs, hamsters, pigs, monkeys or other primates, and gerbils. Agricultural animals include, for example, horses, mules, donkeys, burros, cattle, cows, pigs, sheep, and alligators. Zoo- or circus-maintained animals include, for example, lions, tigers, bears, camels, giraffes, hippopotamuses, and rhinoceroses.

Following are examples that illustrate procedures for practicing the invention. These examples should not be construed as limiting. All percentages are by weight and all solvent mixture proportions are by volume unless otherwise noted.

### Example 1

In one embodiment, blood is mixed with an anticoagulant such as EDTA near or at the time of blood draw. A 0.250 ml blood sample is frozen to a solid then diluted in 0.350 ml water. Alternatively, the blood may be diluted in water first and then frozen. The sample is placed in a 9 X 30 mm Kimball Shell vial #6093114. Two 84-UV-25 collimating lenses are placed on opposite sides of the vial, focused and optionally tiled to about a 10° angle relative to the sample vial. An Ocean Optics QP600-2-SR fiber optic cable (Ocean Optics Inc., Dunedin, FL) is attached to each collimating lens. A Mikropack HPX-2000 high power xenon light source is connected to the lens which is pointing slightly upward to develop the approximate 10° angle adjustment. An Ocean Optics QE 65000 Spectrophotometer (Ocean Optics Inc., Dunedin, FL), set to 100 milliseconds integration time, is connected to the other fiber optic cable attached to the other collimating lens that is optionally slightly pointed down as part of the approximate 10° angle adjustment. The spectrophotometer is run by the Ocean Optics Spectra Suite computer program (Ocean Optics Inc., Dunedin, FL). An Omni*THq* sample homogenizer (Omni International Inc., Marietta, GA) equipped with Omni-Tips (Omni International) is used with the following modification. The plastic barrel of the tip is cut back 2.5 cm allowing only the mixing rotor to emerge into the diluted blood sample.

The Omni Tip homogenizer unit attached to the Omni*THq* driver is immersed in the diluted blood sample and run for 10 seconds at 16,000 rpm. The rotor is then immediately removed and the foam stability is observed as a function of intensity verses nanometers in the Spectra Suite program. The use of a collimating lens for feeding signal to the spectrophotometer, where the radius of the lens is at least half the average distance the foam travels for all samples, allows the Ocean Optics QE 65000 Spectrophotometer to present the rate of foam migration upward as the foam destabilizes while also offering interrogation of the dissolving foam at specific wavelengths.

When the EDTA-treated blood of five bacteria culture positive intensive care unit (ICU) patients was compared to the blood of 5 culture negative donors, the infected patient blood will produce foam where the xenon peak of about 687 nm will take at least 1 minute to reach an intensity level of 60,000 in the Spectra suite program. The same xenon peak will take 55 seconds or less to reach an intensity level of 60,000 when the blood of non-infected patients is tested. The ratio of data points found between 700nm and 755nm will vary depending on whether the patient has a bloodstream infection or if the infection is within an organ such as the bladder. The data points toward the 755nm scale will tend to decrease relative to the data points toward 700 nm when infection is not found in the blood stream (blood culture negative) but is site specific (such as the bladder) positive.

When EDTA blood samples are taken and assayed using the subject method from the indicated culture positive ICU patients twice a day for three days, the results for those initially blood culture positive patients will show a less stable foam (faster time for 687 peak to reach intensity level of 60,000) as the clinical presentation indicates recovery from infection. The foam stability rate (time it takes the foam boundary to migrate upward after agitation as the foam dissolves) is observed to drop steadily over a 24-hour period and over several days. Patients who do not show signs of improvement have corresponding stable foam patterns during those times of poor response to clinical intervention.

### Example 2

In another embodiment, a blood sample (2.0 ml is an example, but smaller or larger volumes may be used) that has first been exposed upon sample draw to an anticoagulant is placed into a vial or other container (*e*.*g*., 7 ml vial, Bio Spec Products Inc., Bartlesville, OK). The sample container is frozen and then thawed. The thawed sample is placed, in one embodiment, into an agitation or shaker device (e.g., a BioSpec Mini Bead Beater 8) and shaken or agitated sufficiently to develop a foam from the entire liquid sample (e.g., for 8 seconds at 3,200 rpm in the Bio Spec Mini Bead Beater). The sample is immediately removed, placed upright, and a timer is immediately started. The time it takes for the foam boundary, formed from shaking, to dissolve and be replaced by the original liquid blood sample is measured. The foam boundary migrates upward as the foam dissolves. The time it takes for the foam boundary to reach any given point is faster in blood from patients who are infected with bacteria or fungi (as evidenced by blood culture) compared to blood from patients who are blood culture negative for infection. In one embodiment, the anticoagulant used in the method is EDTA, SPS, Citrate, Heparin, Sodium Fluoride, or any other anticoagulant.

Any elements or limitations of any invention or embodiment thereof disclosed herein can be combined with any and/or all other elements or limitations (individually or in any combination) or any other invention or embodiment thereof disclosed herein, and all such combinations are contemplated with the scope of the invention without limitation thereto.

### REFERENCES

U.S. Patent No. 4,425,427
Hussain, N. et al. (2008) "The biphasic transmittance waveform: An early marker of sepsis in patients with neutropenia" Thromb Haemost, 100:146-148.
Prata et al. (2008 Oct/Dec) "Composition and physicochemical properties of two protein fractions of bovine serum" Ciênc. Tecnol. Aliment., 28(4).
St. Louis, P. (2007 September) "Markers of Sepsis: A Review" Point of Care: The Journal of Near-Patient Testing & Technology, 6(3):165-169.
Tseng et al. (1997 May) J Magn Reson, 126(1):79-86.
Zakariah, A. (2008) "Combination of biphasic transmittance waveform with blood procalcitonin levels for diagnosis of sepsis in acutely ill patients" Crit Care Med, 36(5).

## Claims

1. A method for determining the presence or absence of a bacterial or fungal infection in a person or animal , the method comprising the steps of a) exposing an anticoagulant-treated blood sample of the person or animal to a freezing temperature wherein the blood is frozen to a solid phase, b) thawing the frozen blood sample to a liquid phase, c) agitating the blood sample to develop foam, and d) measuring the rate that the foam dissolves or dissipates.

2. The method according of claim 1, wherein the rate at which foam dissolves or dissipates is measured by electrical current sensors which are in contact with the blood sample, visually or using acoustic waves.

3. The method according to claim 1, wherein the rate at which foam dissolves or dissipates is measured by a spectrophotometric device.

4. The method according to claim 3, wherein the illumination source of the spectrophotometric device is a xenon lamp.

5. The method according to any one of claims 1 to 4, wherein the blood sample agitation and measurement of the rate at which foam dissolves or dissipates occurs while the blood sample is inside a blood collection device.

6. The method according to any one of claims 1 to 5, wherein agitating the blood sample to develop foam comprises sonication, shaking or introducing of a gas into the blood sample.

7. The method according to any one of claims 1 to 5, wherein agitating the blood sample to develop foam comprises movement of an object that is immersed in the blood sample.

8. A method for determining the presence or absence of a bacterial or fungal infection in a person or animal, the method comprising the steps of a) exposing an anticoagulant-treated blood sample of the person or animal to a freezing temperature in a transparent container wherein the blood is frozen to a solid phase, b) thawing the frozen blood sample to a liquid phase, c) mixing by movement of an object that is immersed in the blood sample in order to develop foam, and d) measuring the rate that the foam dissolves or dissipates by illuminating one side of the transparent blood sample container and measuring the rate foam dissolves or dissipates by capturing transmitted light on the other side of the blood sample container using a collimating lens, wherein the diameter of the collimating lens is at least equal to half of the vertical path traveled by the foam liquid boundary as the foam dissolves or dissipates.

9. The method according to claim 8, wherein the plane transecting the path of both the light from the illumination source and the light entering the collimating lens is tilted at an angle that is greater or less than 90° to the plane of the blood sample container wall.

10. A method according to claim 8, wherein the radius of the collimating lens is at least half the length of the sample container which is occupied by the liquid phase of the blood sample.

11. The method according to claim 8, wherein the illumination source and the light collecting collimating lens which is attached to a light detector are tilted at an angle that is greater or less than 90° relative to the plane of the blood sample container.

12. The method according to any one of claims 8-11, wherein the rate at which foam dissolves or dissipates is measured by a spectrophotometric device.

13. The method according to claim 12, wherein the illumination source of the spectrophotometric device is a xenon lamp.

14. The method according to any one of claims 1 to 13, wherein the anticoagulant is ethylenediaminetetraacetic acid (EDTA), sodium polyanethole sulfonate (SPS), citrate, sodium fluoride or heparin.

15. The method according to any one of claims 1 to 14, wherein the blood sample is diluted with a reagent which contains water.

16. The method according to any one of claims 1 to 15, further comprising determining the level or aggregation of DNA with fibrin.

17. The method according to any one of claims 1 to 16, further comprising comparing the rate of foam dissolution or dissipation of the blood sample to a control rate of foam dissolution or dissipation.

18. The method according to claim 17 wherein the control rate is obtained by treating according to the method of any one of claims 1, 7 or 8 a blood sample from a person or animal that is not infected.

19. The method according to claim 17, wherein the control rate is obtained by contacting a blood sample with DNAse, plasminogen and streptokinase, or synthetic melittin and phospholipase A₂ and treating the contacted blood sample according to the method of any one of claims 1 to 16.

20. A method for monitoring the status or presence of an infection in a person or animal over a period of time, which comprises conducting the method according to any preceding claim, and at least one subsequent time point conducting the method according to any preceding claim, and analysing the results of the methods to determine whether an infection, which may be a bacterial or fungal infection, is present or is persisting or subsiding in the person or animal, wherein an increasing dissolution or dissipation rate over the period of time is indicative that the person or animal is recovering from an infection and wherein a stable or decreasing dissolution or dissipation rate over the period of time is indicative that the person or animal is not recovering from the infection, or that the infection is spreading or getting worse in the person or animal.

21. The method according to any one of claims 1 to 20, wherein the infection, which may be a bacterial or fungal infection, is a bacterial infection.

22. The method according to claim 21, wherein the bacterial infection is from *Staphylococcus, Streptococcus*, *Salmonella, Bacillus, Clostridium, Pseudomonas*, *Neisseria*, *Mycobacterium*, *Yersinia, Haemophilus*, *Bordetella, Listeria*, *Treponema*, *Chlamydia*, *Brucella* or *Vibrio.*

23. The method according to any one of claims 1 to 20, wherein the infection, which may be a bacterial or fungal infection, is a fungal infection.

24. The method according to claim 23, wherein the fungal infection is from *Histoplasma* (e.g. *Histoplasma capoulatum*), *Coccidioides* (e.g. C. *immitis*), *Blastomyces* (e.g.. *B. dermatitidis*), *Candida*, *Aspergillus, Cryptococcus* or *Zygomycetes.*

## Patentansprüche

1. Ein Verfahren zum Bestimmen des Vorhandenseins oder Nichtvorhandenseins einer Bakterien- oder Pilzinfektion bei einer Person oder einem Tier, wobei das Verfahren folgende Schritte umfasst, nämlich a) Aussetzen einer mit einem Antikoagulans behandelten Blutprobe der Person oder des Tiers an eine Minustemperatur, bei der das Blut zu einer festen Phase gefroren wird, b) Auftauen der gefrorenen Blutprobe in eine flüssige Phase, c) Schütteln der Blutprobe, damit sich Schaum bildet und d) Messen der Geschwindigkeit, bei der sich der Schaum auflöst oder dissipiert.

2. Das Verfahren entsprechend Anspruch 1, wobei die Geschwindigkeit, bei der sich der Schaum auflöst oder dissipiert mit Stromsensoren, die mit der Blutprobe in Kontakt sind, visuell oder mit Hilfe von Schallwellen gemessen wird.

3. Das Verfahren entsprechend Anspruch 1, wobei die Geschwindigkeit, bei der sich der Schaum auflöst oder dissipiert mit einem spektrophotometrischen Gerät gemessen wird.

4. Das Verfahren entsprechend Anspruch 3, wobei die Beleuchtungsquelle des spektrophotmetrischen Geräts eine Xenonlampe ist.

5. Das Verfahren entsprechend einem der Ansprüche 1 bis 4, wobei das Schütteln der Blutprobe und das Messen der Geschwindigkeit, bei Schaum sich auflöst oder dissipiert, erfolgt, während sich die Blutprobe in einer Blutentnahmevorrichtung befindet.

6. Das Verfahren entsprechend einem der Ansprüche 1 bis 5, wobei das Schütteln der Blutprobe zwecks Schaumbildung Beschallung, Schütteln oder Zufuhr eines Gases zur Blutprobe umfasst.

7. Das Verfahren entsprechend einem der Ansprüche 1 bis 5, wobei das Schütteln der Blutprobe zur Schaumbildung das Bewegen eines Objekts umfasst, das in der Blutprobe eingetaucht ist.

8. Ein Verfahren zum Bestimmen des Vorhandenseins oder Nichtvorhandenseins einer Bakterien- oder Pilzinfektion bei einer Person oder einem Tier, wobei das Verfahren folgende Schritte umfasst, nämlich a) Aussetzen einer mit einem Antikoagulans behandelten Blutprobe der Person oder des Tiers in einem transparenten Behälter an eine Minustemperatur, bei der das Blut zu einer festen Phase gefroren wird, b) Auftauen der gefrorenen Blutprobe in eine flüssige Phase, c) Mischen durch Bewegen eines in der Blutprobe eingetauchten Objekts, damit sich Schaum bildet und d) Messen der Geschwindigkeit, bei der sich der Schaum auflöst oder dissipiert, indem eine Seite des transparenten Blutprobenbehälters beleuchtet wird und die Geschwindigkeit, bei der sich Schaum auflöst oder dissipiert gemessen wird, indem an die andere Seite des Blutprobenbehälters übertragenes Licht unter Verwendung einer Kollimatorlinse erfasst wird, wobei der Durchmesser der Kollimatorlinse zumindest der Hälfte des vertikalen Wegs entspricht, der von der Schaum-Flüssigkeitsgrenze zurückgelegt wird, wenn sich der Schaum auflöst oder dissipiert.

9. Das Verfahren entsprechend Anspruch 8, wobei die Ebene, die den Weg sowohl des Lichts von der Beleuchtungsquelle als auch des Lichts, das in die Kollimatorlinse fällt, durchschneidet, in einem Winkel von mehr oder weniger als 90° zur Ebene der Wand des Blutprobenbehälters geneigt ist.

10. Ein Verfahren entsprechend Anspruch 8, wobei der Radius der Kollimatorlinse zumindest die Hälfte der Länge des Probenbehälters beträgt, in dem sich die flüssige Phase der Blutprobe befindet.

11. Das Verfahren entsprechend Anspruch 8, wobei die Beleuchtungsquelle und die lichtsammelnde Kollimatorlinse, die an einem Lichtdetektor angebracht ist, in einem Winkel von mehr oder weniger als 90°C relativ zur Ebene des Blutprobenbehälters geneigt sind.

12. Das Verfahren entsprechend einem der Ansprüche 8 - 11, wobei die Geschwindigkeit, bei der sich der Schaum auflöst oder dissipiert mit einem spektrophotometrischen Gerät gemessen wird.

13. Das Verfahren entsprechend Anspruch 12, wobei die Beleuchtungsquelle des spektrophotmetrischen Geräts eine Xenonlampe ist.

14. Das Verfahren entsprechend einem der Ansprüche 1 bis 13, wobei das Antikoagulans Ethylendiamintetraessigsäure (EDTA), Sodium-Polyanethol-Sulfonat (SPS), Zitrat, Natriumfluorid oder Heparin ist.

15. Das Verfahren entsprechend einem der Ansprüche 1 bis 14, wobei die Blutprobe mit einem Reagenz, das Wasser enthält, verdünnt wird.

16. Das Verfahren entsprechend einem der Ansprüche 1 bis 15, das zudem das Bestimmen des DNA-Anteils oder der DNA-Aggregation mit Fibrin gehört.

17. Das Verfahren entsprechend einem der Ansprüche 1 bis 16, zu dem weiterhin das Vergleichen der Geschwindigkeit der Schaumauflösung oder -dissipation der Blutprobe mit einer Kontrollgeschwindigkeit von Schaumauflösung oder -dissipation gehört.

18. Das Verfahren entsprechend Anspruch 17, wobei die Kontrollgeschwindigkeit durch Behandeln einer Blutprobe von einer Person oder einem Tier, die/das nicht infiziert ist, entsprechend dem Verfahren einer Ansprüche 1, 7 oder 8 erhalten wird.

19. Das Verfahren entsprechend Anspruch 17, wobei die Kontrollgeschwindigkeit durch Kontaktieren einer Blutprobe mit DNAse, Plasminogen und Streptokinase oder synthetischem Melittin und Phospholipase A₂ und Behandeln der kontaktierten Blutprobe entsprechend dem Verfahren einer der Ansprüche 1 bis 16 erhalten wird.

20. Ein Verfahren zur Überwachung des Status oder des Vorhandenseins einer Infektion bei einer Person oder einem Tier über einen Zeitraum, das die Durchführung des Verfahrens entsprechend einem der vorhergehenden Ansprüche und bei zumindest einem nachfolgenden Zeitpunkt die Durchführung des Verfahrens entsprechend einem der vorhergehenden Ansprüche und Analyse der Ergebnisse der Verfahren umfasst, um festzustellen, ob eine Infektion, bei der es sich um eine Bakterien- oder Pilzinfektion handeln kann, bei der Person oder dem Tier vorhanden ist oder fortbesteht oder abklingt, wobei eine steigende Auflösungs- oder Dissipationsgeschwindigkeit über den Zeitraum anzeigt, dass sich die Person oder das Tier von einer Infektion erholt, und wobei eine stabile oder sich langsamere Auflösungs- oder Dissipationsgeschwindigkeit über den Zeitraum anzeigt, dass sich die Person oder das Tier nicht von der Infektion erholt oder dass sich die Infektion in der Person oder dem Tier ausbreitet oder verschlimmert.

21. Das Verfahren entsprechend einem der Ansprüche 1 bis 20, wobei die Infektion, bei der er sich um eine Bakterien- oder Pilzinfektion handeln kann, eine Bakterieninfektion ist.

22. Das Verfahren entsprechend Anspruch 21, wobei die Bakterieninfektion durch *Staphylococcus, Streptococcus Salmonella*, *Bacillus*, *Clostridium*, *Pseudomonas*, *Neisseria*, *Mycobacterium*, *Yersinia*, *Haemophilus*, *Bordetella*, *Listeria*, *Treponenia*, *Chlciniydia, Brucella* oder *Vibrio* verursacht wird.

23. Das Verfahren entsprechend einem der Ansprüche 1 bis 20, wobei die Infektion, bei der er sich um eine Bakterien- oder Pilzinfektion handeln kann, eine Pilzinfektion ist.

24. Das Verfahren entsprechend Anspruch 23, wobei die Pilzinfektion durch *Histoplasma* (z.B. *Histoplasma capoulatum*), *Coccidioides* (z.B. C. *immitis*), *Blastomyces* (z.B. *dermatitidis*), *Candida*, *Aspergillus, Cryptococcus* oder *Zygomycetes* verursacht wird.

## Revendications

1. Procédé de détermination de la présence ou de l'absence d'une infection bactérienne ou fongique chez une personne ou un animal, le procédé comprenant les étapes consistant à a) exposer un échantillon de sang traité par un anticoagulant de la personne ou de l'animal à une température de congélation, dans lequel le sang est congelé à une phase solide, b) décongeler l'échantillon de sang à une phase liquide, c) agiter l'échantillon de sang de manière à développer une mousse, et d) mesurer la vitesse de dissolution ou de dissipation de la mousse.

2. Procédé selon la revendication 1, dans lequel la vitesse de dissolution ou de dissipation de la mousse est mesurée par des capteurs de courant électrique qui sont en contact, visuellement ou en utilisant des ondes acoustiques, avec l'échantillon de sang.

3. Procédé selon la revendication 1, dans lequel la vitesse de dissolution ou de dissipation de la mousse est mesurée par un dispositif spectrophotométrique.

4. Procédé selon la revendication 3, dans lequel la source d'éclairage du dispositif spectrophotométrique est une lampe au xénon.

5. Procédé selon l'une quelconque des revendications 1 à 4, dans lequel l'agitation de l'échantillon de sang et la mesure de la vitesse de dissolution ou de dissipation ont lieu tandis que l'échantillon de sang se trouve à l'intérieur du dispositif de collecte de sang.

6. Procédé selon l'une quelconque des revendications 1 à 5, dans lequel l'agitation de l'échantillon de sang pour développer une mousse comprend une sonication, un secouage ou l'introduction d'un gaz dans l'échantillon de sang.

7. Procédé selon l'une quelconque des revendications 1 à 5, dans lequel l'agitation de l'échantillon de sang pour développer une mousse comprend le déplacement d'un objet qui est immergé dans l'échantillon de sang.

8. Procédé de détermination de la présence ou de l'absence d'une infection bactérienne ou fongique chez une personne ou un animal, le procédé comprenant les étapes consistant à a) exposer un échantillon de sang de la personne ou de l'animal traité par un anticoagulant à une température de congélation dans un contenant transparent dans lequel le sang est congelé à une phase solide, b) décongeler l'échantillon de sang congelé à une phase liquide, c) mélanger par déplacement d'un objet qui est immergé dans l'échantillon de sang de manière à développer une mousse, et d) mesurer la vitesse de dissolution ou de dissipation de la mousse en éclairant un côté du contenant de l'échantillon de sang et en mesurant la vitesse de dissolution ou de dissipation en capturant la lumière transmise de l'autre côté du contenant de l'échantillon de sang en utilisant une lentille convergente, le diamètre de la lentille convergente étant au moins égal à la moitié du trajet vertical parcouru par la barrière liquide de confinement de la mousse à mesure que la mousse se dissous ou se dissipe.

9. Procédé selon la revendication 8, dans lequel le plan passant à travers le trajet à la fois de la lumière provenant de la source d'éclairage et la lumière entrant dans la lentille convergente est incliné à un angle qui est supérieur ou inférieur à 90° par rapport au plan de la paroi du contenant de l'échantillon de sang.

10. Procédé selon la revendication 8, dans lequel le rayon de la lentille convergente est au moins de la moitié de la longueur du contenant de l'échantillon qui est occupé par la phase liquide de l'échantillon de sang.

11. Procédé selon la revendication 8, dans lequel la source d'éclairage et la lentille convergente de collecte de lumière qui est fixée au détecteur de lumière sont inclinées à un angle qui est supérieur ou inférieur à 90° relativement au plan du contenant de l'échantillon de sang.

12. Procédé selon l'une quelconque des revendications 8 à 11, dans lequel la vitesse de dissolution ou de dissipation est mesurée par un dispositif spectrophotométrique.

13. Procédé selon la revendication 12, dans lequel la source d'éclairage du dispositif spectrophotométrique est une lampe au xénon.

14. Procédé selon l'une quelconque des revendications 1 à 13, dans lequel l'anticoagulant est de l'acide éthylènediaminetétraacétique (EDTA), du polyanéthole sulfonate de sodium (SPS), du citrate, du fluorure de sodium ou de l'héparine.

15. Procédé selon l'une quelconque des revendications 1 à 14, dans lequel l'échantillon de sang est dilué avec un réactif qui contient de l'eau.

16. Procédé selon l'une quelconque des revendications 1 à 15, comprenant en outre la détermination du niveau ou de l'agrégation de l'ADN avec la fibrine.

17. Procédé selon l'une quelconque des revendications 1 à 16, comprenant en outre la comparaison de la vitesse de dissolution et de dissipation de la mousse de l'échantillon de sang à une vitesse de contrôle de la dissolution ou de la dissipation de la mousse.

18. Procédé selon la revendication 17, dans lequel la vitesse de contrôle est obtenue en traitant un échantillon de sang d'une personne ou d'un animal qui n'est pas infecté selon le procédé de l'une quelconque des revendications 1, 7 ou 8.

19. Procédé selon la revendication 17, dans lequel la vitesse de contrôle est obtenue en mettant en contact un échantillon de sang avec de l'ANDase, un plasmogène et une streptokinase ou une melittine synthétique et de la phospholipase A₂ et en traitant l'échantillon de sang selon le procédé de l'une quelconque des revendications 1 à 16.

20. Procédé de surveillance du statut ou de la présence d'une infection chez une personne ou un animal sur une période de temps, qui comprend l'exécution du procédé selon l'une quelconque des revendications précédentes et à au moins une date ultérieure, exécuter le procédé selon l'une quelconque des revendications précédentes, et analyser les résultats des procédés afin de déterminer si une infection, qui peut être une infection bactérienne ou fongique, est présente ou est persistante ou est en régression chez la personne ou l'animal, dans lequel une vitesse de dissolution ou de dissipation croissante sur une période de temps est indicatrice que la personne ou l'animal est en voie de guérison de l'infection et dans lequel une vitesse de dissolution ou de dissipation stable ou décroissante sur la période de temps est indicatrice que la personne ou l'animal ne se rétablit pas de l'infection, ou que l'infection se propage ou s'aggrave chez la personne ou l'animal.

21. Procédé selon l'une quelconque des revendications 1 à 20, dans lequel l'infection, qui peut être une infection bactérienne ou fongique, est une infection bactérienne.

22. Procédé selon la revendication 21, dans lequel l'infection bactérienne est induite par *Staphylococcus, Streptococcus*, *Saltiionella*, *Bacillus, Clostridium*, *Pseudomonas*, *Neisseria*, *Mycobacterium*, *Yersinia*, *Haemophilus*, *Bordetella*, *Listera*, *Treponemaa*, *Chlamydia*, *Brucella* ou *Vibrio*.

23. Procédé selon l'une quelconque des revendications 1 à 20, dans lequel l'infection, qui peut être une infection bactérienne ou fongique, est une infection fongique.

24. Procédé selon la revendication 23, dans lequel l'infection fongique est induite par *Histoplasma* (par ex. *Hispoplasma Capoulatum*), *Coccidioides* (par ex. C. *immitis*), *Blastomyces* (par ex. *B. dermatitidis*), *Candida, Aspergillus*, *Cryptococcus* ou *Zygomycetes.*
